(19) Europäisches Patentamt
European Patent Office
Office européen des brevets

(11) Veröffentlichungsnummer : **0 071 792**
**B1**

(12) # EUROPÄISCHE PATENTSCHRIFT

(45) Veröffentlichungstag der Patentschrift : **30.01.85**

(21) Anmeldenummer : **82106335.1**

(22) Anmeldetag : **15.07.82**

(51) Int. Cl.[4] : **C 07 D487/04**, A 01 N 43/90 // (C07D487/04, 239/00, 231/00),(C07D487/04, 249/00, 239/00)

(54) **7-Amino-azolo(1,5-a)pyrimidine, Verfahren zu ihrer Herstellung und diese enthaltende Fungizide.**

(30) Priorität : **01.08.81 DE 3130633**

(43) Veröffentlichungstag der Anmeldung : **16.02.83 Patentblatt 83/07**

(45) Bekanntmachung des Hinweises auf die Patenterteilung : **30.01.85 Patentblatt 85/05**

(84) Benannte Vertragsstaaten : **AT BE CH DE FR GB IT LI LU NL SE**

(56) Entgegenhaltungen :
**CHEMICAL ABSTRACTS, Band 63, Nr. 2, 19. Juli 1965, Spalte 1804f, Columbus, Ohio, USA**

(73) Patentinhaber : **BASF Aktiengesellschaft**
**Carl-Bosch-Strasse 38**
**D-6700 Ludwigshafen (DE)**

(72) Erfinder : **Eicken, Karl, Dr.**
**Waldstrasse 63**
**D-6706 Wachenheim (DE)**
Erfinder : **Scheib, Klaus, Dr.**
**Duerkheimer Strasse 7**
**D-6701 Schauernheim (DE)**
Erfinder : **Theobald, Hans, Dr.**
**Parkstrasse 2**
**D-6703 Limburgerhof (DE)**
Erfinder : **Pommer, Ernst-Heinrich, Dr.**
**Berliner Platz 7**
**D-6703 Limburgerhof (DE)**
Erfinder : **Ammermann, Eberhard, Dr.**
**Sachsenstrasse 3**
**D-6700 Ludwigshafen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 071 792 B1

**Beschreibung**

Die Erfindung betrifft neue 7-Amino-azolo [1,5-a] pyrimidine, Verfahren zu ihrer Herstellung und diese enthaltende Fungizide.

Es ist bekannt, daß 7-Amino-azolo [1,5-a] pyrimidine, z. B. das 7-Amino-2-methyl-5-phenyl-pyrazolo [1,5-a] pyrimidin, pharmakologische Eigenschaften besitzen (FR-PS 2 448 542 ; DD-PS 99 794 ; DD-PS 55 956 ; J. pharm. Soc. Japan *84* (1964), S. 1113-1118). Es ist ferner bekannt, N-Trichlor-methylthiophthalimid als Fungizid zu verwenden (Chemical Week 1972, June 21, Seite 63).

Es wurde nun gefunden, daß neue 7-Amino-azolo [1,5-a] pyrimidine der Formel

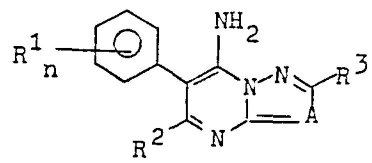

(I)

worin

$R^1$ gegebenenfalls durch Halogen oder $C_1$-$C_4$-Alkoxi substituiertes $C_1$-$C_{12}$-Alkyl, Halogen, $C_1$-$C_{12}$-Alkoxi, Cyano, $C_3$-$C_8$-Cycloalkyl, Phenyl, Phenyloxi, Phenylthio, Benzyl, Benzyloxi, Benzylthio, mit dem Phenylring annelliertes Benzol, Indan oder Tetrahydronaphthalin, welche gegebenenfalls im aromatischen Teil durch $C_1$-$C_4$-Alkyl, $C_1$-$C_4$-Alkoxi, Halogen oder Cyano substituiert sind,

n 1 oder 2

$R^2$ und $R^3$ Wasserstoff, $C_1$-$C_4$-Alkyl, oder Phenyl, gegebenenfalls substituiert durch Chlor, $C_1$-$C_4$-Alkyl oder $C_1$-$C_4$-Alkoxi,

A ein Stickstoffatom oder eine $CR^4$-Gruppe bedeuten, wobei

$R^4$ die Bedeutung von $R^2$ hat und zusätzlich Halogen, Cyano oder $C_1$-$C_4$-Alkoxicarbonyl oder zusammen mit $R^3$ eine $C_3$-$C_4$-Alkylenkette gegebenenfalls mit bis zu 2 Doppelbindungen bedeutet,

eine gute fungizide Wirkung, insbesondere gegen Phycomyceten haben.

Unter den Resten $R^1$ sind beispielsweise gegebenenfalls durch Fluor, Chlor, Brom oder $C_1$-$C_4$-Alkoxi substituiertes $C_1$-$C_{12}$-Alkyl, Fluor, Chlor, Brom, $C_1$-$C_{12}$-Alkoxi, Cyano, $C_3$-$C_8$-Cycloalkyl, Phenyl, Phenyloxi, Phenylthio, Benzyl, Benzyloxi, Benzylthio, mit dem Phenylring annelliertes Benzol, Indan oder Tetrahydronaphthalin, welche gegebenenfalls im aromatischen Teil durch $C_1$-$C_4$-Alkyl, $C_1$-$C_4$-Alkoxi, Cyano, Fluor, Chlor oder Brom substituiert sein können, zu verstehen.

Unter den Resten $R^2$, $R^3$ und $R^4$ in der Bedeutung von $R^2$ ist beispielsweise Wasserstoff, $C_1$-$C_4$-Alkyl oder gegebenenfalls durch Chlor, $C_1$-$C_4$-Alkyl oder $C_1$-$C_4$-alkoxi substituiertes Phenyl zu verstehen. Darüber hinaus kann $R^4$ Chlor, Brom, Cyan oder $C_1$-$C_4$-alkoxicarbonyl bedeuten oder zusammen mit $R^3$ eine gegebenenfalls bis zu zwei Doppelbindungen enthaltende $C_3$-$C_4$-Alkylenkette bedeuten. Unter Alkyl oder Alkyl einer Alkoxigruppe bei den Resten $R^1$, $R^2$, $R^3$ und $R^4$ ist je nach Zahl der angegebenen Kohlenstoffatome Methyl, Ethyl, Propyl, Butyl, Pentyl, Hexyl, Heptyl, Octyl, Nonyl, Decyl, Undecyl oder Dodecyl und ihre Isomeren zu verstehen.

Es wurde ferner gefunden, daß man 7-Amino-azolo [1,5-a] pyrimidine der Formel I erhält, indem man substituierte Benzylcyanide der Formel

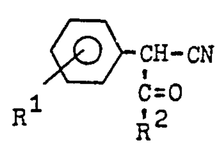

(II)

in welcher $R^1$ und $R^2$ die oben angegebenen Bedeutungen haben, mit 5(3)-Aminopyrazolen der Formel

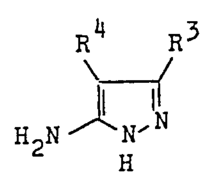

(III)

oder mit 5(3)-Amino-1,2,4-triazolen der Formel

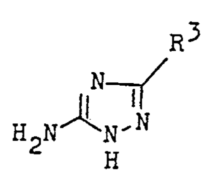

(IV)

in welcher $R^3$ und $R^4$ die oben angegebenen Bedeutungen haben, umsetzt.

Die Umsetzung kann in Gegenwart oder Abwesenheit von Lösungsmitteln durchgeführt werden. Vorteilhaft ist es, solche Lösungsmittel zu verwenden, gegenüber denen die Einsatzstoffe weitgehend inert sind und in denen sie ganz oder teilweise löslich sind. Als Lösungsmittel kommen insbesondere Alkohole wie Ethanol, Propanole, Butanole, Glykole oder Glykolmonoether, Diethylenglykole oder deren Monoether, Amide wie Dimethylformamid, Diethylformamid, Dibutylformamid, N,N-Dimethylacetamid, niedere Alkansäuren wie Ameisensäure, Essigsäure, Propionsäure und Mischungen dieser Lösungsmittel mit Wasser in Frage. Die Umsetzungstemperaturen liegen zwischen 50 und 300 °C, vorzugsweise bei 50 bis 150 °C, wenn in Lösung gearbeitet wird.

Die neuen 7-Amino-azolo [1,5-a] pyrimidine werden gegebenenfalls nach Verdampfen des Lösungsmittels oder Verdünnen mit Wasser als kristalline, meist sehr reine Verbindungen isoliert. Bei Verwendung von niederen Alkansäuren als Lösungsmitteln ist es zweckmäßig, gegebenenfalls nach teilweisem Verdampfen der Alkansäure, die Reste der Alkansäure durch Zugabe von wäßrigem Alkali zu neutralisieren, wobei die neuen 7-Amino-azolo [1,5-a] pyrimidine meist in sehr reiner Form auskristallisieren.

Die für die Herstellung der 7-Amino-azolo [1,5-a] pyrimidine benötigten substiuierten Benzylcyanide der Formel

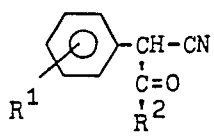

(II)

sind teilweise bekannt oder können nach bekannten Methoden aus Benzylcyaniden und Carbonsäureestern mit Alkalialkoholaten oder Alkalihydriden hergestellt werden [J. Amer. Chem. Soc. *73,* (1951) S. 3766].

Allgemeine Herstellungsvorschrift für die substituierten Benzylcyanide der Formel II :

1,5 Mol Natriumalkoholat wird in 1 l Toluol eingetragen und anschließend 1,0 Mol eines Benzylcyanids und dann 2,0 Mol eines Carbonsäureesters unter Rühren zugetropft, wobei die Temperatur auf 40 bis 50 °C ansteigt. Nach 2-stündigem Nachrühren bei 75 bis 80 °C wird abgekühlt und mit 2 l Wasser versetzt. Aus der wäßrigen Phase isoliert man nach zweimaligem Waschen mit 0,2 l Toluol durch Ansäuern mit halbkonzentrierter (etwa 50 Gew.-%) Schwefelsäure auf pH 2 das substituierte Benzylcyanid der Formel II (Ausbeuten : 70 bis 90 %).

Auf diese Weise können folgende substituierten Benzylcyanide der Formel

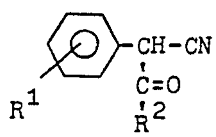

(II)

hergestellt werden :

| $R^1$ | $R^2$ | Fp. (°C) |
|---|---|---|
| $2-CH_3$ | H | 89 |
| $3-CH_3$ | H | 119 |
| $4-C(CH_3)_3$ | H | 169 |
| $3-CH_3O$ | H | 102 |
| 3-Cl | H | 178 |
| 4-Cl | H | 164 |
| 4-Br | H | 176 |
| $3-CF_3$ | H | 107 |
| $3-CF_3$ | $CH_3$ | 82 |
| $3-C_6H_5O$ | H | 45 |
| $4-C_2H_5$ | H | 90 |
| $4-C_6H_{13}O$ | H | 116 |
| $4-iC_3H_7$ | H | 84 |

(Fortsetzung)

| $R^1$ | $R^2$ | Fp. (°C) |
|---|---|---|
| 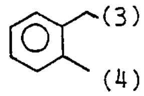 | H | 205 |
| 3,4-$Cl_2$ | H | 170 |
| 2-$CH_3$, 4-$C(CH_3)_3$ | H | 120 |
| 4-$C_6H_5$ | H | 228 |
| 4-$C_6H_5CH_2$-O | H | 188 |
| 4 ($ClCH_2CH(CH_3)CH_2$) | H | Öl |
| 2,4-$Cl_2$ | H | 166 |
| 4-CN | H | 222 |
| 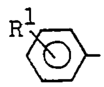 = ß-Naphthyl | H | |
| 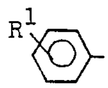 = α-Naphthyl | H | |

Die folgenden Beispiele betreffen die Herstellung der neuen Wirkstoffe.

## Beispiel 1

21,3 g m-Trifluormethyl-2-formyl-benzylcyanid und 9,7 g 3(5)-Amino-5(3)-methylpyrazol wurden in 100 ml Eisessig 4 Stunden am Rückfluß erhitzt. Nach dem Abkühlen wurde der Ansatz mit 500 ml Wasser verdünnt und mit 2 n NaOH-Lösung auf pH 5 bis 6 eingestellt, wobei ein öliges Produkt ausfiel, das nach Anreiben kristallisierte. Nach Absaugen der Kristalle, mehrfachen Waschen mit Wasser und Trocknen im Vakuum bei 50 °C erhielt man 25,0 g 7-Amino-2-methyl-6-(3'-trifluormethylphenyl) pyrazolo [1,5-a] pyrimidin vom Fp. 176 °C (Verbindung 10).

$C_{14}H_{11}N_4F_3$ (M 292)
ber.: C 57,54 H 3,79 N 19,17
gef.: C 57,6 H 3,9 N 18,9

## Beispiel 2

10,5 g p-tert.-Butyl-2-formyl-benzylcyanid und 4,8 g 3(5)-Amino-5(3)-methylpyrazol wurden in 40 ml Dimethylformamid 3 Stunden am Rückfluß erhitzt. Nach dem Abkühlen wurden 150 ml Wasser zugetropft. Nach dem Absaugen der Kristalle, Waschen mit Wasser und Trocknen im Vakuum bei 50 °C erhielt man 11,3 g 7-Amino-2-methyl-6-(4'-tert. butylphenyl) pyrazolo [1,5-a] pyrimidin vom Fp. 218 °C (Verbindung 5).

$C_{17}H_{20}N_4$ (M 280)
ber.: C 72,83 H 7,19 N 19,98
gef.: C 72,8 H 7,1 N 19,9

## Beispiel 3

11,8 g m-Phenoxi-2-formylbenzylcyanid und 4,3 g 3-Aminotriazol wurden in 40 ml Eisessig 6 Stunden am Rückfluß erhitzt, nach dem Abkühlen mit 300 ml Wasser versetzt und mit 2 n NaOH auf pH 6 eingestellt. Die ausgefallenen Kristalle wurden abgesaugt und getrocknet (14,1 g). Nach Lösen in 30 ml heißem Dimethylformamid abkühlen, Fällen mit 10 ml Methanol, Waschen der abgesaugten Kristalle mit weiterem Methanol und Trocknen erhielt man 9,6 g 7-Amino-6-(3'-phenoxiphenyl)-1,2,4-triazolo [1,5-a] pyrimidin vom Fp. 248-250 °C (Verbindung 44).

$C_{17}H_{13}N_5O$ (M 303)
ber.: C 67,32 H 4,32 N 23,09
gef.: C 67,8 H 4,2 N 22,9

Nach den oben beschriebenen Verfahren wurden folgende 7-Amino-azolo [1,5-a] pyrimidine hergestellt.

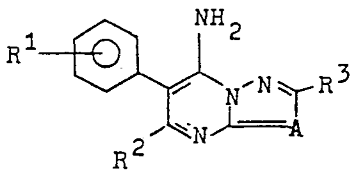

| Nr. | $R^1$ | $R^2$ | $R^3$ | $R^4$ | A | Fp. (°C) |
|---|---|---|---|---|---|---|
| 1 | $3-CF_3$ | $CH_3$ | $CH_3$ | H | $CR^4$ | 212 |
| 2 | $3,4(CH_3O)_2$ | $CH_3$ | $CH_3$ | H | $CR^4$ | 188 |
| 3 | $2-CH_3$ | H | $CH_3$ | H | $CR^4$ | 224 |
| 4 | $3-CH_3$ | H | $CH_3$ | H | $CR^4$ | 158 |
| 5 | $4-C(CH_3)_3$ | H | $CH_3$ | H | $CR^4$ | 218 |
| 6 | $3-CH_3O$ | H | $CH_3$ | H | $CR^4$ | 124 |
| 7 | 3-Cl | H | $CH_3$ | H | $CR^4$ | 174 |
| 8 | 4-Cl | H | $CH_3$ | H | $CR^4$ | 168 |
| 9 | 4-Br | H | $CH_3$ | H | $CR^4$ | 171 |
| 10 | $3-CF_3$ | H | $CH_3$ | H | $CR^4$ | 176 |
| 11 | $3-C_6H_5O$ | H | $CH_3$ | H | $CR^4$ | 173 |
| 12 | $4-C_2H_5$ | H | $CH_3$ | H | $CR^4$ | 150 |
| 13 | $4-H_{13}C_6O$ | H | $CH_3$ | H | $CR^4$ | 132 |
| 14 | 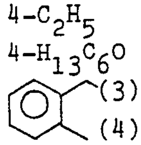 | H | $CH_3$ | H | $CR^4$ | 328 |
| 15 | $4-iC_3H_7$ | H | $CH_3$ | H | $CR^4$ | 162 |
| 16 | $3,4-Cl_2$ | H | $CH_3$ | H | $CR^4$ | 160 |
| 17 | $4-C(CH_3)_3$; $2-CH_3$ | H | $CH_3$ | H | $CR^4$ | 238 |
| 18 | $4-C_6H_5$ | H | $CH_3$ | H | $CR^4$ | 197 |
| 19 | $4-C_6H_5-CH_2O$ | H | $CH_3$ | H | $CR^4$ | 160 |
| 20 | $4-(ClCH_2CH(CH_3)CH_2)$ | H | $CH_3$ | H | $CR^4$ | 168 |
| 21 | $2,4-Cl_2$ | H | $CH_3$ | H | $CR^4$ | 245 |
| 22 | $3-CF_3$ | H | H | $C_6H_5$ | $CR^4$ | 184 |
| 23 | $3-CF_3$ | H | CH=CH-CH=CH | | $CR^4$ | 243 |
| 24 | $4-C(CH_3)_3$ | H | CH=CH-CH=CH | | $CR^4$ | 248 |
| 25 | $4-CH_3O$ | $CH_3$ | $CH_3$ | H | $CR^4$ | 200 |
| 26 | $3-C_6H_5O$ | H | H | H | $CR^4$ | 166 |
| 27 | $4-C(CH_3)_3$ | H | H | H | $CR^4$ | 210 |
| 28 | $3-CF_3$ | $CH_3$ | H | $CO_2CH_3$ | $CR^4$ | 273 |
| 29 | $3-CF_3$ | $CH_3$ | H | $CO_2C_2H_5$ | $CR^4$ | 196 |
| 30 | $4-C(CH_3)_3$ | H | H | $C_6H_5$ | $CR^4$ | 231 |
| 31 | 4-CN | H | $CH_3$ | H | $CR^4$ | 229 |
| 32 | $4-C(CH_3)_3$ | H | $CH_3$ | Br | $CR^4$ | 258 |
| 34 | 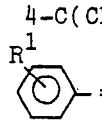 = ß-Naphthyl | H | $CH_3$ | H | $CR^4$ | 242 |
| 35 | 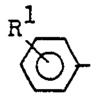 = α-Naphthyl | H | $CH_3$ | H | $CR^4$ | 211 |

(Fortsetzung)

| Nr. | $R^1$ | $R^2$ | $R^3$ | $R^4$ | A | Fp. (°C) |
|---|---|---|---|---|---|---|
| 36 | 2-$CH_3$ | H | H | - | N | 252 |
| 37 | 3-$CH_3$ | H | H | - | N | 222 |
| 38 | 3-$CH_3O$ | H | H | - | N | 246 |
| 39 | 3-$CF_3$ | H | H | - | N | 280 |
| 41 | 4-$C(CH_3)_3$ | H | H | - | N | 327 |
| 42 | 3-Cl | H | H | - | N | 282 |
| 43 | 4-Br | H | H | - | N | 303 |
| 44 | 3-$C_6H_5O$ | H | H | - | N | 250 |
| 45 | 4-Cl | H | H | - | N | 257 |
| 46 | 4-$C_2H_5$ | H | H | - | N | 268 |
| 47 | 4-$C(CH_3)_3$; 2-$CH_3$ | H | H | - | N | 288 |
| 48 | 4-$C_6H_5$ | H | H | - | N | 300 |
| 49 | 4-$H_{13}C_6$-O | H | H | - | N | 256 |
| 50 | 4-i-$C_3H_7$ | H | H | - | N | 272 |
| 51 | 3,4-$Cl_2$ | H | H | - | N | 284 |
| 52 | 2,4-$Cl_2$ | H | H | - | N | 283 |
| 53 | 4($ClCH_2CH(CH_3)CH_2$) | H | H | - | N | 217 |
| 54 | 4-$C_6H_5CH_2$-O | H | H | - | N | 268 |
| 55 | 4-CN | H | H | - | N | 345 |
| 57 | 4-$C(CH_3)_3$ | H | $C_6H_5$ | - | N | 370 |
| 58 | 4-$C(CH_3)_3$ | $CH_3$ | $CH_3$ | H | $CR^4$ | 242 |
| 59 | 4-$C(CH_3)_3$ | $C_2H_5$ | $CH_3$ | H | $CR^4$ | 168 |
| 60 | 4-$C(CH_3)_3$ | n-$C_3H_7$ | $CH_3$ | H | $CR^4$ | 192 |
| 61 | 4(4'-$C(CH_3)_3$-$C_6H_4CH_2O$) | H | $CH_3$ | H | $CR^4$ | 207 |
| 62 | 4-$C(CH_3)_3$ | H | $CH_3$ | CN | $CR^4$ | 300 |
| 63 | 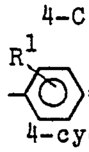 =beta-Naphthyl | H | H | - | N | 201 |
| 64 | 4-cycl.$C_6H_{11}$ | H | $CH_3$ | H | $CR^4$ | 200 |
| 65 | 4-$C(CH_3)_3$ | $CH_3$ | $CH_3$ | Br | $CR^4$ | 260 |
| 66 | 4-$C_2H_5O$ | $CH_3$ | $CH_3$ | H | $CR^4$ | 218 |
| 67 | 4-$C_2H_5O$ | H | H | - | N | 258 |
| 68 | 4-$C_2H_5O$ | H | $CH_3$ | H | $CR^4$ | 185 |
| 69 | 4-$C_2H_5O$ | $CH_3$ | H | - | N | 202 |
| 70 | 4-n$H_{13}C_6O$ | $CH_3$ | $CH_3$ | H | $CR^4$ | 168 |
| 71 | 4($CH_2$=CH-$CH_2O$) | H | H | - | N | 235 |
| 72 | 4($CH_2$=CH-$CH_2O$) | H | $CH_3$ | H | $CR^4$ | 161 |
| 73 | 4(n-$C_4H_9$-CH($C_2H_5$)-$CH_2O$) | H | $CH_3$ | H | $CR^4$ | 102 |
| 74 | 4(n-$C_4H_9$-CH($C_2H_5$)-$CH_2O$) | H | H | - | N | 199 |
| 75 | 4(n-$C_{12}H_{25}O$) | H | $CH_3$ | H | $CR^4$ | 98 |
| 76 | 4(n-$C_{12}H_{25}O$) | H | H | - | N | 198 |
| 77 | 4(n-$C_4H_9O$) | H | $CH_3$ | H | $CR^4$ | 181 |
| 78 | 4(n-$C_4H_9O$) | H | H | - | N | 235 |
| 79 | 4(i-$C_4H_9O$) | H | $CH_3$ | H | $CR^4$ | 211 |
| 80 | 4(i-$C_4H_9O$) | H | H | - | N | 270 |

In entsprechender Weise können die folgenden Verbindungen hergestellt werden :

| Nr. | $R^1$ | $R^2$ | $R^3$ | $R^4$ | A | Fp. (°C) |
|---|---|---|---|---|---|---|
| 33 | $4-C(CH_3)_3$ | H | $-(CH_2)_3-$ | | | |
| 40 | $3-CF_3$ | $CH_3$ | H | - | N | |
| 56 | $4-C(CH_3)_3$ | $CH_3$ | H | - | | |

Die neuen Wirkstoffe zeigen eine starke fungitoxische Wirksamkeit gegen phytopathogene Pilze, insbesondere aus der Klasse der Phycomyceten. Die neuen Verbindungen sind daher beispielsweise geeignet zur Bekämpfung von Phytophthora infestans an Tomaten und Kartoffeln, Phytophthora parasitica an Erdbeeren, Phytophthora cactorum an Äpfeln, Pseudoperonospora cubensis an Gurken, Pseudoperonospora humuli an Hopfen, Peronospora destructor an Zwiebeln, Peronospora sparsa an Rosen, Peronospora tabacina an Tabak, Plasmopara viticola an Reben, Plasmopara halstedii an Sonnenblumen, Sclerospora macrospora an Mais, Bremia lactucae an Salat, Mucor mucedo an Früchten, Rhizopus nigricans an Rüben, Erysiphe graminis an Getreide, Uncinula necator an Reben, Podophaera leucotricha an Äpfeln, Sphaerotheca fuliginea an Rosen, Erysiphe cichoriacearum an Gurken. Die fungiziden Mittel enthalten 0,1 bis 95 % (Gewichtsprozent) Wirkstoff, vorzugsweise 0,5 bis 90 %. Die Aufwandmengen liegen je nach Art des gewünschten Effektes zwischen 0,1 und 5 kg Wirkstoff je ha.

Die neuen Wirkstoffe können auch zusammen mit anderen Wirkstoffen, z. B. Herbiziden, Insektiziden, Wachstumsregulatoren und Fungiziden oder auch mit Düngemitteln vermischt und ausgebracht werden. In vielen Fällen erhält man bei der Mischung mit Fungiziden auch eine Vergrößerung des fungiziden Wirkungsspektrums ; bei einer Anzahl dieser Fungizidmischungen treten auch synergistische Effekte auf, d. h. die fungizide Wirksamkeit des Kombinationsproduktes ist größer als die der addierten Wirksamkeiten der Einzelkomponenten.

Die neuen Wirkstoffe werden beispielsweise in Form von direkt versprühbaren Lösungen, Pulvern, Suspensionen, auch hochprozentige wäßrige, ölige oder sonstige Suspensionen oder Dispersionen, Emulsionen, Öldispersionen, Pasten, Stäubemitteln, Streumitteln, Granulaten, durch Versprühen, Vernebeln, Verstäuben, Verstreuen, Beizen oder Gießen angewendet. Die Aufwendungsformen richten sich ganz nach den Verwendungszwecken ; sie sollten in jedem Fall möglichst die feinste Verteilung der neuen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle, sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z. B. Dimethylformamid, Dimethylsulfoxid, N-Methylpyrrolidon oder Wasser in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen Lösungsmittel gelöst, mittels Haft-, Netz-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind. Als oberflächenaktive Stoffe kommen in Betracht :

Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Poloxyethylenoctylphenylether, ethoxyliertes Isooctylphenol-, Octylphenol-, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether ethoxyliertes Polyoxypropylen, Laurylakoholpolyglykoletheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulaten, können durch Bindung der Wirkstoffe an feste Trägerstoffe hergestellt werden. Feste Trägerstoffe sind z. B. Mineralerden wie Silicagel, Kieselsäuren, Kreide, Talkum, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehle, Baumrinden-, Holz- und Nußschalenmehle, Cellulosepulver und andere feste Trägerstoffe.

Für die folgenden Versuche wurden als bekannte Vergleichswirkstoffe die folgenden Verbindungen verwendet.

N-Trichlormethylthio-phthalimid (Verbindung A), 7-Amino-2-methyl-5-phenyl-pyrazolo [1,5-a] pyrimidin (Verbindung B).

Versuch 1

Wirksamkeit gegen Plasmopara viticola

Blätter von Topfreben der Sorte « Müller-Thurgau » wurden mit wäßriger Spritzbrühe, die 80 % (Gew.-%) Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 10 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 16 Stunden in einer wasserdampfgesättigten Kammer bei 24 °C und anschließend für 8 Tage in einem Gewächshaus mit Temperaturen zwischen 20 und 30 °C aufgestellt. Nach dieser Zeit wurden die Pflanzen zur Beschleunigung des Sporangienträgerausbruchs abermals für 16 Stunden in der feuchten Kammer aufgestellt. Dann erfolgte die Beurteilung des Ausmaßes des Pilzausbruches auf den Blattunterseiten. Beispielsweise zeigten die Wirkstoffe 1, 5, 10, 11, 12, 13, 15, 16, 17, 18, 19, 20, 27, 37, 41, 42, 44 bei Anwendung einer 0,025 %igen Wirkstoffbrühe eine bessere fungizide Wirkung (beispielsweise 100 %ige Wirkung) als die bekannten Vergleichsmittel A und B (beispielsweise 60 %ige Wirkung).

Beispiele für Zubereitungen sind :

I. Man vermischt 90 Gew.-Teile der Verbindung 1 mit 10 Gew.-Teilen N-Methyl-alpha-pyrrolidon und erhält eine Lösung, die zur Anwendung in Form kleinster Tropfen geeignet ist.

II. 20 Gew.-Teile der Verbindung 5 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamin, 5 Gew.-Teilen Calciumsalz der Dodecylbenzolsulfonsäure und 5 Gew.-Teilen des Anlagerungsproduktes von 40 Mol Ethylenoxid an 1 Mol Rizinusöl besteht. Durch Ausgießen und feines Verteilen der Lösung in Wasser erhält man eine wäßrige Dispersion.

III. 20 Gew.-Teile der Verbindung 10 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Rizinusöl besteht. Durch Eingießen und feines Verteilen der Lösung in Wasser erhält man eine wäßrige Dispersion.

IV. 20 Gew.-Teile der Verbindung 11 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid and 1 Mol Rizinusöl besteht. Durch Eingießen und feines Verteilen der Lösung in Wasser erhält man eine wäßrige Dispersion.

V. 80 Gew.-Teile der Verbindung 37 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in der Mischung in Wasser erhält man eine Spritzbrühe.

VI. 3 Gew.-Teile der Verbindung 41 werden mit 97 Gew.-Teilen feinteiligem Kaolin innig vermischt. Man erhält auf diese Weise ein Stäubemittel, das 3 Gew.-% des Wirkstoffs enthält.

VII. 30 Gew.-Teile der Verbindung 42 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung 44 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit Wasser erhält man eine wäßrige Dispersion.

IX. 20 Teile der Verbindung 1 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölig Dispersion.

**Ansprüche** (für die Vertragsstaaten : BE, CH, DE, FR, GB, IT, LI, LU, NL, SE)

1. 7-Amino-azolo [1,5-a] pyrimidine der Formel

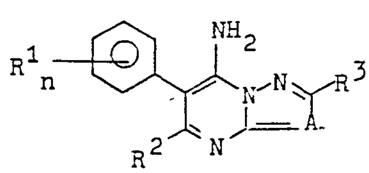

(I)

worin

$R^1$ gegebenenfalls durch Halogen oder $C_1$-$C_4$-Alkoxi substituiertes $C_1$-$C_{12}$-Alkyl, Halogen, $C_1$-$C_{12}$-Alkoxi, Cyano, $C_3$-$C_8$-Cycloalkyl, Phenyl, Phenyloxi, Phenylthio, Benzyl, Benzyloxi, Benzylthio, mit dem Phenylring annelliertes Benzol, Indan oder Tetrahydronaphthalin, welche gegebenenfalls im aromatischen Teil durch $C_1$-$C_4$-Alkyl, $C_1$-$C_4$-Alkoxi, Halogen oder Cyano substituiert sind,

n 1 oder 2

$R^2$ und $R^3$ Wasserstoff, $C_1$-$C_4$-Alkyl, oder Phenyl, gegebenenfalls substituiert durch Chlor, $C_1$-$C_4$-Alkyl oder $C_1$-$C_4$-Alkoxi,

A ein Stickstoffatom oder eine $CR^4$-Gruppe bedeuten, wobei

$R^4$ die Bedeutung von $R^2$ hat und zusätzlich Halogen, Cyano oder $C_1$-$C_4$-Alkoxicarbonyl oder zusammen mit $R^3$ eine $C_3$-$C_4$-Alkylenkette gegebenenfalls mit bis zu 2 Doppelbindungen bedeutet.

2. Fungizid enthaltend ein 7-Amino-azolo [1,5-a] pyrimidin der Formel I gemäß Anspruch 1.

3. Fungizid, enthaltend einen festen oder flüssigen Trägerstoff und ein 7-Amino-azolo [1,5-a] pyrimidin der Formel I gemäß Anspruch 1.

4. Verfahren zur Herstellung eines Fungizids, dadurch gekennzeichnet, daß man einen festen oder flüssigen Trägerstoff vermischt mit einem 7-Amino-azolo [1,5-a] pyrimidin der Formel I gemäß Anspruch 1.

5. Verfahren zur Bekämpfung von phytopathogenen Pilzen, dadurch gekennzeichnet, daß man die Pilze oder die vor Pilzbefall zu schützenden Gegenstände mit einem 7-Amino-azolo [1,5-a] pyrimidin der Formel I gemäß Anspruch 1 behandelt.

6. Verfahren zur Herstellung eines 7-Amino-azolo [1,5-a] pyrimidins der Formel I gemäß Anspruch 1, dadurch gekennzeichnet, daß man ein substituiertes Benzylcyanid der Formel

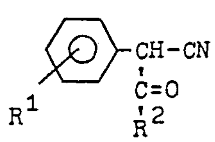

(II)

in der $R^1$ und $R^2$ die im Anspruch 1 genannte Bedeutung haben, mit einem 5(3)-Aminopyrazol der Formel

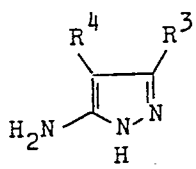

(III)

oder mit einem 5(3)-Amino-1,2,4-triazol der Formel

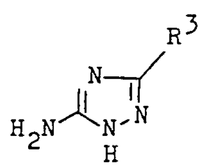

(IV)

in welcher $R^3$ und $R^4$ die im Anspruch 1 angegebenen Bedeutungen haben, umsetzt.

7. 7-Amino-azolo [1,5-a] pyrimidin der Formel I gemäß Anspruch 1, dadurch gekennzeichnet, daß $R^2$ Wasserstoff oder Methyl, $R^3$ Wasserstoff oder Methyl und A CH bedeuten.

8. Fungizid, enthaltend ein 7-Amino-azolo [1,5-a] pyrimidin, der Formel I gemäß Anspruch 1, dadurch gekennzeichnet, daß $R^2$ Wasserstoff oder Methyl, $R^3$ Wasserstoff oder Methyl und A CH bedeuten.

**Ansprüche** (für den Vertragsstaat AT)

1. Fungizid, enthaltend ein 7-Amino-azolo [1,5-a] pyrimidin der Formel

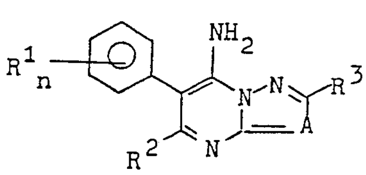

(I)

worin

$R^1$ gegebenenfalls durch Halogen oder $C_1$-$C_4$-Alkoxi substituiertes $C_1$-$C_{12}$-Alkyl, Halogen, $C_1$-$C_{12}$-Alkoxi, Cyano, $C_3$-$C_8$-Cycloalkyl, Phenyl, Phenyloxi, Phenylthio, Benzyl, Benzyloxi, Benzylthio, mit dem Phenylring annelliertes Benzol, Indan oder Tetrahydronaphthalin, welche gegebenenfalls im aromatischen Teil durch $C_1$-$C_4$-Alkyl, $C_1$-$C_4$-Alkoxi, Halogen oder Cyano substituiert sind,

n 1 oder 2

$R^2$ und $R^3$ Wasserstoff, $C_1$-$C_4$-Alkyl, oder Phenyl, gegebenenfalls substituiert durch Chlor, $C_1$-$C_4$-Alkyl oder $C_1$-$C_4$-Alkoxi,

A ein Stickstoffatom oder eine $CR^4$-Gruppe bedeuten, wobei

$R^4$ die Bedeutung von $R^2$ hat und zusätzlich Halogen, Cyano oder $C_1$-$C_4$-Alkoxicarbonyl oder zusammen mit $R^3$ eine $C_3$-$C_4$-Alkylenkette gegebenenfalls mit bis zu 2 Doppelbindungen bedeutet.

2. Fungizid, enthaltend einen festen oder flüssigen Trägerstoff und ein 7-Amino-azolo [1,5-a] pyrimidin der Formel I gemäß Anspruch 1.

3. Verfahren zur Herstellung eines Fungizids, dadurch gekennzeichnet, daß man einen festen oder flüssigen Trägerstoff vermischt mit einem 7-Amino-azolo [1,5-a] pyrimidin der Formel I gemäß Anspruch 1.

4. Verfahren zur Bekämpfung von phytopathogenen Pilzen, dadurch gekennzeichnet, daß man die Pilze oder die vor Pilzbefall zu schützenden gegenstände mit einem 7-Amino-azolo [1,5-a] pyrimidin der Formel I gemäß Anspruch 1 behandelt.

5. Verfahren zur Herstellung eines 7-Amino-azolo [1,5-a] pyrimidins der Formel I gemäß Anspruch 1, dadurch gekennzeichnet, daß man ein substituiertes Benzylcyanid der Formel

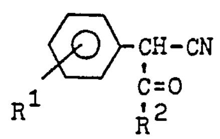

(II)

,

in der $R^1$ und $R^2$ die im Anspruch 1 genannte Bedeutung haben, mit einem 5(3)-Aminopyrazol der Formel

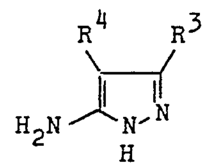

(III)

oder mit einem 5(3)-Amino-1,2,4-triazol der Formel

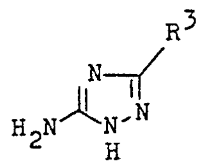

(IV)

in welcher $R^3$ und $R^4$ die im Anspruch 1 angegebenen Bedeutungen haben, umsetzt.

6. Fungizid, enthaltend ein 7-Amino-azolo [1,5-a] pyrimidin, definiert wie in Anspruch 1, dadurch gekennzeichnet, daß $R^2$ Wasserstoff oder Methyl, $R^3$ Wasserstoff oder Methyl und A CH bedeuten.

**Claims** (for the Contracting States : BE, CH, DE, FR, GB, IT, LI, LU, NL, SE)

1. A 7-aminoazolo [1,5-a] pyrimidine of the formula

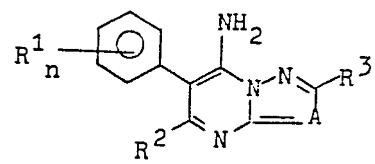

(I)

where

$R^1$ is unsubstituted, halogen-substituted or $C_1$-$C_4$-alkoxy-substituted alkyl of 1 to 12 carbon atoms, halogen, alkoxy of 1 to 12 carbon atoms, cyano, or cycloalkyl of 3 to 8 carbon atoms, or is phenyl, phenoxy, phenylthio, benzyl, benzoxy or benzylthio, each of which may be substituted by $C_1$-$C_4$-alkyl, $C_1$-

$C_4$-alkoxy, halogen or cyano, or is benzene, indane, tetrahydronaphthalene, each of which may be substituted in the aromatic moiety by $C_1$-$C_4$-alkyl, $C_1$-$C_4$-alkoxy, halogen or cyano and each of which is fused to the phenyl ring,

n is 1 or 2,

$R^2$ and $R^3$ are each hydrogen, $C_1$-$C_4$-alkyl or phenyl which is unsubstituted or substituted by chlorine, $C_1$-$C_4$-alkyl or $C_1$-$C_4$-alkoxy, and

A is nitrogen or a $CR^4$ group, where

$R^4$ has the meanings of $R^2$ and also denotes halogen, cyano or alkoxycarbonyl, or together with $R^3$ is alkylene which may have up to two double bonds.

2. A fungicide containing a 7-aminoazolo [1,5-a] pyrimidine of the formula I as claimed in claim 1.

3 A fungicide containing a solid or liquid carrier and a 7-aminoazolo [1,5-a] pyrimidine of the formula I as claimed in claim 1.

4. A process for producing a fungicide, wherein a solid or liquid carrier is mixed with a 7-aminoazolo [1,5-a] pyrimidine of the formula I as claimed in claim 1.

5. A process for combating phytopathogenic fungi, wherein the fungi or the objects to be protected against fungus attack are treated with a 7-aminoazolo [1,5-a] pyrimidine as claimed in claim 1.

6. A process for preparing a 7-aminoazolo [1,5-a] pyrimidine of the formula I as claimed in claim 1, wherein a substituted benzyl cyanide or the formula

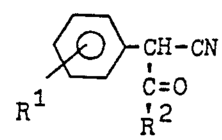

(II)

where $R^1$ and $R^2$ have the meanings given in claim 1, is reacted with a 5(3)-aminopyrazole of the formula

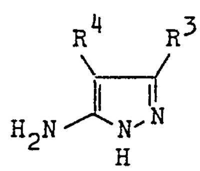

(III)

or with a 5(3)-amino-1,2,4-triazole of the formula

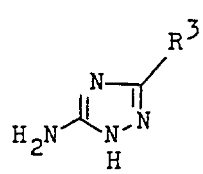

(IV)

where $R^3$ and $R^4$ have the meanings given in claim 1.

7. A 7-aminoazolo [1,5-a] pyrimidine of the formula I as claimed in claim 1, where $R^2$ is hydrogen or methyl, $R^3$ is hydrogen or methyl, and A is CH.

8. A fungicide containing a 7-aminoazolo [1,5-a] pyrimidine of the formula I as claimed in claim 1, where $R^2$ is hydrogen or methyl, $R^3$ is hydrogen or methyl, and A is CH.

**Claims** (for the Contracting State AT)

1. A fungicide containing a 7-aminoazolo [1,5-a] pyrimidine of the formula

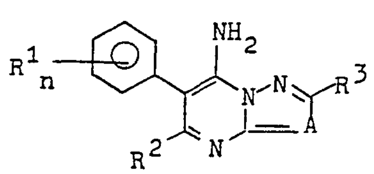

(I)

where

$R^1$ is unsubstituted, halogen-substituted or $C_1$-$C_4$-alkoxy-substituted alkyl of 1 to 12 carbon atoms, halogen, alkoxy of 1 to 12 carbon atoms, cyano, or cycloalkyl of 3 to 8 carbon atoms, or is phenyl, phenoxy, phenylthio, benzyl, benzoxy or benzylthio, each of which may be substituted by $C_1$-$C_4$-alkyl, $C_1$-

$C_4$-alkoxy, halogen or cyano, or is benzene, indane, tetrahydronaphthalene, each of which may be substituted in the aromatic moiety by $C_1$-$C_4$-alkyl, $C_1$-$C_4$-alkoxy, halogen or cyano and each of which is fused to the phenyl ring,

n is 1 or 2,

$R^2$ and $R^3$ are each hydrogen, $C_1$-$C_4$-alkyl or phenyl which is unsubstituted or substituted by chlorine, $C_1$-$C_4$-alkyl or $C_1$-$C_4$-alkoxy, and

A is nitrogen or a $CR^4$ group, where

$R^4$ has the meanings of $R^2$ and also denotes halogen, cyano or alkoxycarbonyl, or together with $R^3$ is alkylene which may have up to two double bonds.

2. A fungicide containing a solid or liquid carrier and a 7-aminoazolo [1,5-a] pyrimidine of the formula I as defined in claim 1.

3. A process for producing a fungicide, wherein a solid or liquid carrier is mixed with a 7-aminoazolo [1,5-a] pyrimidine of the formula I as defined in claim 1.

4. A process for combating phytopathogenic fungi, wherein the fungi or the objects to be protected against Tungus attack are treated with a 7-aminoazolo [1,5-a] pyrimidine as defined in claim 1.

5. A process for preparing a 7-aminoazolo [1,5-a] pyrimidine of the formula I as claimed in claim 1, wherein a substituted benzyl cyanide of the formula

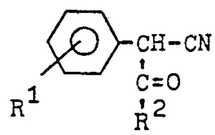

(II)

where $R^1$ and $R^2$ have the meanings given in claim 1, is reacted with a 5(3)-aminopyrazole of the formula

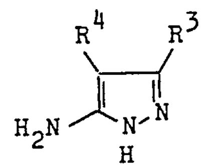

(III)

or with a 5(3)-amino-1,2,4-triazole of the formula

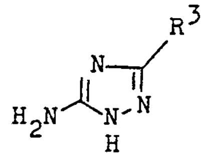

(IV)

where $R^3$ and $R^4$ have the meanings given in claim 1.

6. A fungicide containing a 7-aminoazolo [1,5-a] pyrimidine of the formula I as defined in claim 1, where $R^2$ is hydrogen or methyl, $R^3$ is hydrogen or methyl, and A is CH.

**Revendications** (pour les Etats contractants : BE, CH, DE, FR, GB, IT, LI, LU, NL, SE)

1. 7-Amino-azolo [1,5-a] pyrimidines de formule

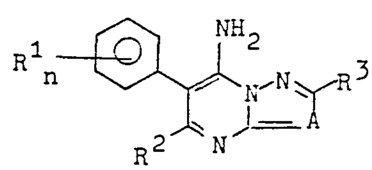

(I)

dans laquelle

$R^1$ représente alkyle en $C_1$-$C_{12}$, éventuellement substitué par halogène ou alcoxy en $C_1$-$C_4$, halogène, alcoxy en $C_1$-$C_{12}$, cyano, cycloalkyle en $C_3$-$C_8$, phényle, phényloxy, phénylthio, benzyle, benzyloxy, benzylthio, benzène condensé avec le noyau phényle, indane ou tétrahydronaphtalène, qui sont éventuellement substitués, dans la partie aromatique, par alkyle en $C_1$-$C_4$, alcoxy en $C_1$-$C_4$ halogène ou cyano,

n est 1 ou 2

$R^2$ et $R^3$ représentent hydrogène, alkyle en $C_1$-$C_4$ ou phényle, éventuellement substitués par chlore, alkyle en $C_1$-$C_4$ ou alkoxy en $C_1$-$C_4$

A représente un atome d'azote ou un groupe $CR^4$

$R^4$ ayant la signification de $R^2$ et, en outre, représentant halogène, cyano ou alcoxycarbonyle en $C_1$-$C_4$, ou bien, avec $R^3$, une chaîne alkylène en $C_3$-$C_4$, éventuellement avec jusqu'à 2 doubles liaisons.

2. Fongicide contenant une 7-amino-azolo [1,5-a] pyrimidine de formule I selon la revendication 1.

3. Fongicide contenant un support solide ou liquide et une 7-amino-azolo [1,5-a] pyrimidine de formule I selon la revendication 1.

4. Procédé de préparation d'un fongicide, caractérisé par le fait qu'on mélange un support solide ou liquide avec une 7-amino-azolo [1,5-a] pyrimidine de formule I selon la revendication 1.

5. Procédé de lutte contre les champignons phytopathogènes, caractérisé par le fait que l'on traite les champignons, ou les objets à protéger contre l'attaque des champignons, avec une 7-amino-azolo [1,5-a] pyrimidine de formule I selon la revendication 1.

6. Procédé de préparation d'une 7-amino-azolo [1,5-a] pyrimidine de formule I selon la revendication 1, caractérisé par le fait qu'on fait réagir un cyanure de benzyle substitué de formule

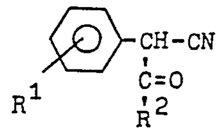

(II)

dans laquelle $R^1$ et $R^2$ ont les significations indiquées dans la revendication 1, avec une 5(3)-aminopyrazole de formule

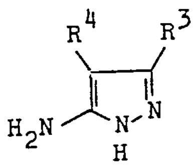

(III)

ou avec une 5(3)-amino-1,2,4-triazole de formule

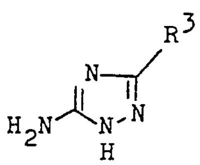

(IV)

dans laquelle $R^3$ et $R^4$ ont les significations données dans la revendication 1.

7. 7-amino-azolo [1,5-a] pyrimidine de formule I selon la revendication 1, caractérisé par le fait que $R^2$ représente hydrogène ou méthyle, $R^3$ hydrogène ou méthyle et A représente CH.

8. Fongicide contenant une 7-amino-azolo [1,5-a] pyrimidine de formule I selon la revendication 1, caractérisé par le fait que $R^2$ représente hydrogène ou méthyle, $R^3$ hydrogène ou méthyle et A représente CH.

**Revendications** (pour l'Etat contractant AT)

1. Fongicide contenant une 7-amino-azolo [1,5-a] pyrimidine de formule

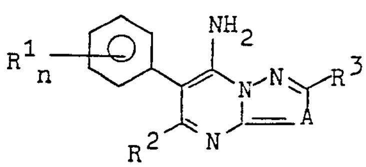

(I)

dans laquelle

$R^1$ représente alkyle en $C_1$-$C_{12}$, éventuellement substitué par halogène ou alcoxy en $C_1$-$C_4$, halogène, alcoxy en $C_1$-$C_{12}$, cyano, cycloalkyle en $C_3$-$C_8$, phényle, phényloxy, phénylthio, benzyle, benzyloxy, benzylthio, benzène condensé avec le noyau phényle, indane ou tétrahydronaphtalène, qui

sont éventuellement substitués, dans la partie aromatique, par alkyle en $C_1$-$C_4$, alcoxy en $C_1$-$C_4$ halogène ou cyano,

n est 1 ou 2

$R^2$ et $R^3$ représentent hydrogène, alkyle en $C_1$-$C_4$ ou phényle, éventuellement substitués par chlore, alkyle en $C_1$-$C_4$ ou alkoxy en $C_1$-$C_4$

A représente un atome d'azote ou un groupe $CR^4$

$R^4$ ayant la signification de $R^2$ et, en outre, représentant halogène, cyano ou alcoxycarbonyle en $C_1$-$C_4$, ou bien, avec $R^3$, une chaîne alkylène en $C_3$-$C_4$, éventuellement avec jusqu'à 2 doubles liaisons.

2. Fongicide contenant un support solide ou liquide et une 7-amino-azolo [1,5-a] pyrimidine de formule I selon la revendication 1.

3. Procédé de préparation d'un fongicide, caractérisé par le fait qu'on mélange un support solide ou liquide avec une 7-amino-azolo [1,5-a] pyrimidine de formule I selon la revendication 1.

4. Procédé de lutte contre les champignons phytopathogènes, caractérisé par le fait que l'on traite les champignons, ou les objets à protéger contre l'attaque des champignons, avec une 7-amino-azolo [1,5-a] pyrimidine de formule I selon la revendication 1.

5. Procédé de préparation d'une 7-amino-azolo [1,5-a] pyrimidine de formule I selon la revendication 1, caractérisé par le fait qu'on fait réagir un cyanure de benzyle substitué de formule

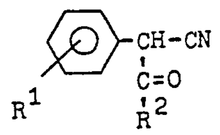

(II)

dans laquelle $R^1$ et $R^2$ ont les significations indiquées dans la revendication 1, avec une 5(3)-aminopyrazole de formule

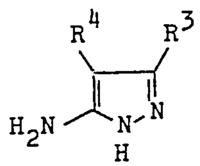

(III)

ou avec une 5(3)-amino-1,2,4-triazole de formule

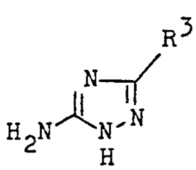

(IV)

dans laquelle $R^3$ et $R^4$ ont les significations données dans la revendication 1.

6. Fongicide contenant une 7-amino-azolo [1,5-a] pyrimidine de formule I selon la revendication 1, caractérisé par le fait que $R^2$ représente hydrogène ou méthyle, $R^3$ hydrogène ou méthyle et A représente CH.